# EUROPEAN PATENT APPLICATION

(11) **EP 1 935 985 A1**
(43) Date of publication of application: **25.06.2008**
(21) Application number: 06810346.4
(22) Date of filing: 13.09.2006
(51) Int. Cl.: C12Q 1/25, G01N 33/60, G21H 5/02

(54) **METHOD OF DETECTING REACTIVE METABOLITE**

(30) Priority: 14.09.2005 JP 2005267637
(71) Applicant: BANYU PHARMACEUTICAL CO., LTD., Tokyo 102-8667 (JP)
(72) Inventor: TAKAHASHI, Hiroyuki, Tsukuba-shi, Ibaraki 300-2611 (JP); INOUE, Kazuko, Tsukuba-shi, Ibaraki 300-2611 (JP); SHIBATA, Yoshihiro, Tsukuba-shi, Ibaraki 300-2611 (JP); CHIBA, Masato, Tsukuba-shi, Ibaraki 300-2611 (JP); ISHII, Yasuyuki, Tsukuba-shi, Ibaraki 300-2611 (JP)
(74) Representative: Buchan, Gavin MacNicol
(86) International application number: PCT/JP2006/318654
(87) International publication number: WO 2007/032544

(57) **Abstract**

A method of detecting a reactive metabolite which comprises steps of conducting a metabolic reaction of a test compound in the presence of two labeled nucleophiles, and detecting the label of the nucleophiles bound to the reactive metabolite, and a method of detecting a reactive metabolite which further comprises separating liquid layer using ammonium acetate to obtain an organic layer after the aforementioned reaction step are disclosed. In these detection methods, a reactive metabolite can be quantitatively detected by easy preparation. Furthermore, generation of a false positive signal can be prevented, and generation of a false negative signal can be decreased. Accordingly, screening of a test compound can be efficiently performed with higher accuracy.

## Description

### Technical Field

The present invention relates to a method of detecting a reactive metabolite that causes drug toxicity.

### Background Art

Recently, it has become important to obtain knowledge on pharmacokinetics and safety of a compound at a relatively early stage of drug discovery research, along with rapid development of screening technology and compound synthesis represented by combinatorial chemistry.

Production of a reactive intermediate that irreversibly inhibits cytochrome P450 known as a metabolic enzyme or covalent binding of a reactive intermediate not only to a metabolic enzyme but also to another protein is often recognized. Since these phenomena may cause late toxicity or teratogenicity, covalent binding is measured in some cases of searching for safer compounds.

A method of measuring covalent binding has been already reported (Non-Patent Document 1). In the method, a labeled test compound is incubated with liver microsomes and subjected to aftertreatment using a Brandel tissue harvester to isolate precipitated protein. The labeled test compound bound to the protein is detected and quantitatively measured. (Non-Patent Document 1)

In addition, a method of trapping a reactive intermediate by using a low-molecular trapping agent is applied. This method is qualitative analysis in which a reactive intermediate derived from a test compound metabolized by a metabolic enzyme is trapped by the low-molecular trapping agent, and the label of this trapping agent is detected. For example, Non-Patent Document 2 and Patent Document 1 disclose a method using labeled glutathione as a tracer and detecting a complex of a reactive intermediate metabolized by liver microsomes with glutathione. Non-Patent Document 3 discloses a method using labeled cyanide as a tracer and detecting a complex of a reactive intermediate metabolized by liver microsomes with cyanide.

Furthermore, Non-Patent Document 4 discloses an assay in which liver microsomes and acetaminophen were incubated in the presence of unlabeled glutathione and cyanide, and a metabolic product is detected using glutathione as a tracer.
Patent Document 1: Japanese Patent Laid-Open No. 2002-238597
Non-Patent Document 1: Chem. Res. Toxicol., 2004, vol. 17, p.3
Non-Patent Document 2: Chemico-Biological Interaction, 1995, vol. 97, p.37
Non-Patent Document 3: Drug Metabolism and Disposition, 1982, vol. 10, 6 p.90
Non-Patent Document 4: Life Sciences, 1991, vol. 48, p.2423

### Disclosure of the Invention

However, the method using a labeled test compound requires synthesis of a radiolabeled test compound and thus has problems that it takes much labor and time. Furthermore, the method of detecting a reaction intermediate using a low-molecular trapping agent as a tracer can be used as a qualitative assay, but is not sufficiently verified for the quantitative determination of the results.

The present invention has been made in view of the problems in the aforementioned conventional technology, and an object of the present invention is to provide a method of detecting a reactive metabolite by easy preparation and with excellent quantitativeness and qualitativeness.

The present inventors have conducted intensive studies to achieve the above-mentioned object and, as a result, have found the fact that a reactive metabolite can be quantitatively detected by conducting a metabolic reaction of a test compound in the presence of two types of labeled low-molecular trapping agents and detecting the reactive metabolite bound to the trapping agents. The present invention has been thus accomplished.

That is, a method of detecting a reactive metabolite according to the present invention comprises steps of conducting a metabolic reaction of a test compound in the presence of two labeled nucleophiles and detecting the label of the nucleophiles bound to the reactive metabolite. With such a method, a reactive metabolite can be simply detected without individually labeling each test compound.

Furthermore, a method of detecting a reactive metabolite according to the present invention comprises steps of conducting a metabolic reaction of a test compound in the presence of two labeled nucleophiles, separating liquid layer using a high-concentration salt solution to obtain an organic layer, and detecting the label of the nucleophiles bound to the reactive metabolite contained in the organic layer. With such a method, a reactive metabolite can be simply detected without individually labeling each test compound, and generation of a false positive (label is detected despite of the absence of covalent binding) or false negative (label is not detected despite of the presence of covalent binding) signal can be prevented.

In the above method of detecting a reactive metabolite according to the present invention, the high-concentration salt is preferably ammonium acetate, sodium acetate, or ammonium formate. With the use of such a salt, generation of a false positive or false negative signal can be prevented.

In addition, the above-described method of detecting a reactive metabolite may further comprise mixing unlabeled cysteine with the organic layer obtained in the separation step and incubating the mixture. Through such a step, generation of a false positive or false negative signal can be further prevented.

In addition, in the above-described method of detecting a reactive metabolite according to the present invention, the two nucleophiles are preferably a soft nucleophile and a hard nucleophile. With the use of such a combination of the nucleophiles, generation of a false positive or false negative signal can be further prevented. Here, cysteine is an example of the soft nucleophile, and cyanide is an example of the hard nucleophile.

Furthermore, in the above-described method of detecting a reactive metabolite according to the present invention, the label is preferably a radioisotope.

Furthermore, a method of detecting a reactive metabolite according to the present invention comprises steps of conducting a metabolic reaction of a test compound in the presence of labeled cysteine and detecting the label of the nucleophile bound to the reactive metabolite. With such a method, a reactive metabolite can be simply detected without individually labeling each test compound.

Furthermore, a method of detecting a reactive metabolite according to the present invention comprises conducting a metabolic reaction of a test compound in the presence of labeled cysteine, separating liquid layer using a high-concentration salt solution to obtain an organic layer, and detecting the label of the nucleophile bound to the reactive metabolite contained in the organic layer. With such a method, a reactive metabolite can be simply detected without individually labeling each test compound, and generation of a false positive or false negative signal can be prevented.

In the above method of detecting a reactive metabolite according to the present invention, the high-concentration salt is preferably ammonium acetate, sodium acetate, or ammonium formate. With the use of such a salt, generation of a false positive or false negative signal can be prevented.

Furthermore, the aforementioned method of detecting a reactive metabolite may further comprise mixing unlabeled cysteine with the organic layer obtained in the separation step and incubating the mixture. Through such a step, generation of a false positive or false negative signal can be further prevented.

According to the method of detecting a reactive metabolite of the present invention, a reaction system can be simply prepared, and a reactive metabolite can be quantitatively and qualitatively detected. In addition, generation of a false positive signal can be prevented, and generation of a false negative signal can be decreased. Consequently, screening for a test compound can be efficiently performed with higher accuracy.

### Brief Description of the Drawings

**Figure 1** is a graph showing a relationship between an RI area and covalent binding evaluated using a Brandel tissue harvester when the covalent binding is detected using [³⁵S]cysteine alone as the trapping agent.
**Figure 2** is a graph showing a relationship between an RI area and covalent binding evaluated using a Brandel tissue harvester when the covalent binding is detected using sodium [¹⁴C]cyanide alone as the trapping agent.
**Figure 3** is a graph showing a relationship between an RI area and covalent binding evaluated using a Brandel tissue harvester when the covalent binding is detected using [³⁵S]cysteine and sodium [¹⁴C]cyanide as the trapping agents;
**Figure 4** is a graph showing a relationship between an RI area and covalent binding evaluated using a Brandel tissue harvester when the covalent binding is detected using [³⁵S]cysteine and sodium [¹⁴C]cyanide as the trapping agents and using an ammonium acetate buffer solution for extraction.
**Figure 5** is a graph showing a result of RI-HPLC (Radioisotope-High performance liquid chromatography) measurement of a metabolized compound.
**Figure 6** is a graph showing a result of MS/MS measurement of a metabolic product.
**Figure 7** is a graph showing a result of MS/MS/MS measurement of the metabolic product.

### Best Mode for Carrying Out the Invention

Exemplary embodiments of the present invention will now be described in detail.

First, the terms employed in the present invention will be described.

The term "reactive metabolite" employed in the present invention means a chemically reactive metabolite (reactive intermediate) that is produced as a result of *in vivo* metabolism of an administered organic compound (mainly a drug or a drug candidate compound).

The term "nucleophile" employed in the present invention means a reaction agent having an electron pair that is involved in the attack of a substrate in a substitution reaction, addition reaction, or the like. The nucleophile is an agent having a high affinity to a reaction center with a shortage of electrons, and, in general, most of them have a lone pair. Furthermore, the terms "soft" and "hard" in the respective "soft nucleophile" and "hard nucleophile" employed in the present invention indicate the "soft" and "hard" in the HSAB (Hard and Soft Acids and Bases) principle, and are terms based on the principle that in Lewis acids and Lewis bases, hard acids prefer to bind to hard bases, and soft acids prefer to bind to soft bases. Examples of the "soft nucleophile" include glutathione, N-acetylcysteine, and cysteine, and examples of a "soft electrophile" include epoxide, quinone, and α,β-unsaturated carbonyl. Examples of the "hard nucleophile" include cyanide (for example, sodium cyanide and potassium cyanide), and examples of a "hard electrophile" include an iminium ion.

The nucleophile according to the present invention is necessarily labeled in a detectable form. Any label can be used without particular limitation, and examples thereof include a radioisotope label and a fluorescent label. The labeling of the nucleophile can also be performed by any method without particular limitation and can be performed by a method known to those in the art.

The "test compound" according to the present invention can be any type of compound without particular limitation, and examples thereof include single compounds such as natural compounds, organic compounds, inorganic compounds, proteins, and peptides, and also compound libraries, expression products of gene libraries, cell extracts, cell cultured supernatants, products of fermented microbes, marine organism extracts, plant extracts, procaryotic cell extracts, eucaryotic cell extracts, and animal cell extracts.

A method of detecting a reactive metabolite according to a first embodiment of the present invention will now be described.

The method of detecting a reactive metabolite according to the first embodiment of the present invention comprises steps of conducting a metabolic reaction of a test compound in the presence of two labeled nucleophiles and detecting the label of the nucleophiles bound to the reactive metabolite.

First, in the reaction step according to the present invention, a metabolic reaction of a test compound is conducted in the presence of two labeled nucleophiles.

It is possible to detect a reactive metabolite using one type of nucleophile in the metabolic reaction of this step. However, in such a case, a signal derived from a specific reactive metabolite may not be detected due to the physical and chemical properties of the nucleophile (false negative). The present inventors make it possible to conduct much accurate assay by using different two nucleophiles and detecting a complementary signal generated by reciprocal properties. In a case that one type of nucleophile is used, cysteine is preferred.

Conditions for the reaction are not particularly limited as long as the metabolic reaction of a test compound occurs, and those that reflect *in vivo* conditions are preferred. Such conditions are, for example, a buffer solution (for example, a phosphate buffer solution or a tris-hydrochloric acid buffer solution) with a pH of 6.0 to 8.0 and a temperature of 35°C to 40°C, and such a solution can be used as the reaction solution. The metabolic reaction is performed by adding a test compound and a metabolic enzyme to the aforementioned solution, and other optional reagents (for example, magnesium chloride) may be added for optimizing reaction conditions. Furthermore, the metabolic enzyme used here may be a specific drug-metabolizing enzyme (for example, cytochrome P450) purified or roughly purified according to the purpose of an assay or an *in vivo* extract (for example, liver microsome) containing a metabolic enzyme.

Furthermore, the test compound, the metabolic enzyme, and so on may be added at the same time as a reaction solution is prepared or after preincubation (for example, for 5 to 30 minutes) of a prepared reaction solution. The metabolic reaction time is not particularly limited and may be arbitrarily determined according to the purpose of an assay.

The reaction is terminated after the lapse of a desired reaction time. The method for terminating the reaction is not particularly limited. For example, the reaction may be terminated by addition of acetonitrile or change of reaction temperature (for example, by standing on ice).

Furthermore, after such aforementioned termination of the reaction, a reaction metabolite bound to the nucleophiles may be subjected to an extraction step, if necessary, for being supplied to the subsequent step. The extraction can be performed by a method known to those in the art. For example, the extraction is performed by addition of acetonitrile and centrifugation, or repeating these steps, to separate an acetonitrile layer and subsequent concentration and drying of the separated acetonitrile layer.

After such a reaction step, detection of the label of the nucleophiles bound to the reactive metabolite is conducted. The detection of the label of the nucleophiles may be performed by an arbitrary method depending on the type of the label. For example, a radioisotope label can be detected by RI-HPLC.

The thus detected nucleophiles have a reactive metabolite bound thereto. That is, the reactive metabolite can be qualitatively and quantitatively determined by such detection.

Next, a method of detecting a reactive metabolite according to a second embodiment of the present invention will be described.

The method of detecting a reactive metabolite according to the second embodiment of the present invention comprises steps of conducting a metabolic reaction of a test compound in the presence of two labeled nucleophiles, separating liquid layer using ammonium acetate to obtain an organic layer, and detecting the label of the nucleophiles bound to the reactive metabolite contained in the organic layer.

First, in the reaction step according to the present invention, a metabolic reaction of a test compound is conducted in the presence of two labeled nucleophiles.

It is possible to detect a reactive metabolite using one type of nucleophile in the metabolic reaction of this step. However, in such a case, a signal derived from a specific reactive metabolite may not be detected due to the physical and chemical properties of the nucleophile (false negative). The present inventors make it possible to conduct much accurate assay by using different two nucleophiles and detecting a complementary signal generated by reciprocal properties. In a case that one type of nucleophile is used, cysteine is preferred.

Conditions for the reaction are not particularly limited as long as the metabolic reaction of a test compound occurs, and those that reflect *in vivo* conditions are preferred. Such conditions are, for example, a buffer solution (for example, a phosphate buffer solution or a tris-hydrochloric acid buffer solution) with a pH of 6.0 to 8.0 and a temperature of 35°C to 40°C, and such a solution can be used as the reaction solution. The metabolic reaction is performed by adding a test compound and a metabolic enzyme to the aforementioned solution, and other optional reagents (for example, magnesium chloride) may be added for optimizing reaction conditions. Furthermore, the metabolic enzyme used here may be a specific drug-metabolizing enzyme (for example, cytochrome P450) that is purified or roughly purified according to the purpose of an assay, or an *in vivo* extract (for example, liver microsome) containing a metabolic enzyme.

Furthermore, the test compound, the metabolic enzyme, and so on may be added at the same time as a reaction solution is prepared or after preincubation (for example, for 5 to 30 minutes) of a prepared reaction solution. The metabolic reaction time is not particularly limited and may be arbitrarily determined according to the purpose of an assay.

The reaction is terminated after the lapse of a desired reaction time. The method of terminating the reaction is not particularly limited. For example, the reaction may be terminated by addition of acetonitrile or change of reaction temperature (for example, by standing on ice).

Furthermore, after the reaction step, a step of extracting a reaction metabolite bound to the nucleophiles may be performed, according to need. The extraction can be performed by a method known to those in the art. For example, the extraction may be performed by addition of acetonitrile and centrifugation, or repeating these steps, to separate an acetonitrile layer and subsequent concentration and drying of the separated acetonitrile layer.

Then, the solution after the termination of the reaction is subjected to liquid layer separation using a high-concentration salt solution to obtain an organic layer. Here, examples of the high-concentration salt include ammonium acetate, sodium acetate, and ammonium formate. Furthermore, in a case using sodium acetate or ammonium formate, the high-concentration salt solution preferably has a concentration of 0.05 to 5.0 M, more preferably 0.1 to 2 M, further preferably 0.5 to 1.5 M, and particularly preferably 0.7 to 1.3 M. In a case using ammonium acetate, the concentration is preferably 0.1 to 2 M, more preferably 0.5 to 1.5 M, and further preferably 0.7 to 1.3 M. The addition amount may be arbitrarily determined according to the total amount of a reaction system. For example, the high-concentration salt solution of the same amount as that of the reaction system may be used. With the addition of the high-concentration salt, a structure that causes false positive can be decomposed. As a result, generation of false positive can be decreased. For example, the present inventors have found that, in a case of a test compound having a thiazolidine structure in the molecule, the thiazolidine structure can be decomposed by the addition of ammonium acetate to decrease false positive.

Then, unlabeled (cold) cysteine is added to the organic layer, and the mixture is incubated. With this step, false positive can be prevented or significantly decreased. The concentration of cysteine is not particularly limited and is preferably 10 to 60 mM. In addition, any material that is substitutable for labeled cysteine can be used instead of the aforementioned unlabeled cysteine, and an example of such a material is glutathione.

If a test compound generates a false positive signal, though covalent binding does not occur, namely, the test compound does not have reactivity and is thought not to be toxic to a living body in this point, the signal leads to the contrary conclusion and causes rejection of a compound having favorable properties as a drug. However, such disadvantage can be avoided by using an ammonium acetate solution or unlabeled cysteine in the separation step, and the discovery of a drug can be more efficiently performed.

After the separation step, detection of the label of the nucleophiles bound to the reactive metabolite is conducted. The detection of the label of the nucleophiles may be performed by an arbitrary method depending on the type of the label. For example, a radioisotope label can be detected by RI-HPLC. When qualitative analysis is performed, MS (mass spectrometry) may be simultaneously performed. The measurement by RI-HPLC and MS may be conducted by a method known to those in the art and can be conducted according to the manual attached to a device to be used.

The thus detected nucleophiles have a reactive metabolite bound thereto. That is, the reactive metabolite can be qualitatively and quantitatively determined by such detection.

As described above, according to the method of detecting a reactive metabolite of the present invention, a test compound is not required to be labeled, and therefore a reaction system can be simply prepared. Furthermore, a reactive metabolite can be qualitatively and also quantitatively detected. In addition, generation of a false positive or false negative signal can be prevented, and, consequently, screening for a test compound can be efficiently performed with higher accuracy.

### Examples

The present invention will hereinafter be more specifically described with reference to examples, but is not limited thereto.

### Example 1

### Incubation

A phosphate buffer solution (100 mM, pH 7.4) containing liver microsomes (1 mg/mL), 3 mM of magnesium chloride, 50 µM of test compound A, and 1 mM of [³⁵S]cysteine or 100 µM of sodium [¹⁴C]cyanide, as the final concentrations, was preincubated at 37°C for 5 minutes, and then an NADPH aqueous solution was added therein to reach the final concentration of 1 mM to start the reaction (incubation volume: 150 µL). One hour after the start of the incubation, acetonitrile (450 µL: a volume three times the volume of the reaction solution) was added to the reaction solution to terminate the reaction. Then, 150 µL of a phosphate buffer solution (1 M) containing 50 mM ofnon-radiolabeled cysteine was added to the solution. The resulting mixture was sufficiently mixed at room temperature and was then subjected to centrifugation at 10000 g for 5 minutes at room temperature to give an acetonitrile layer. Liquid-liquid extraction was performed again using 450 µL of acetonitrile as in above to give an acetonitrile layer. The resulting acetonitrile solution was concentrated and dried under nitrogen stream, and the residue was resuspended in 40 µL of a 40% methanol aqueous solution for being subjected to RI-HPLC analysis.

### HPLC analysis

Thirty microliters of the sample prepared by the aforementioned incubation was injected into RI-HPLC. To prevent an RI-detector cell from being contaminated with foreign substances, elimination was conducted using a switching valve on the RI-HPLC side of the RI detector for five minutes after the injection. HPLC conditions were as follows:
Model: Agilent1100 series
Column temperature: 40°C
Mobile phase:
   Binary pump A: CH₃CN/100 mM AcONH₄/H₂O = 5/10/85 (V/V/V)
   Binary pump B: CH₃CN/100 mM AcONH₄ = 90/10 (V/V)
The analysis was conducted by 75-minute gradient elution.

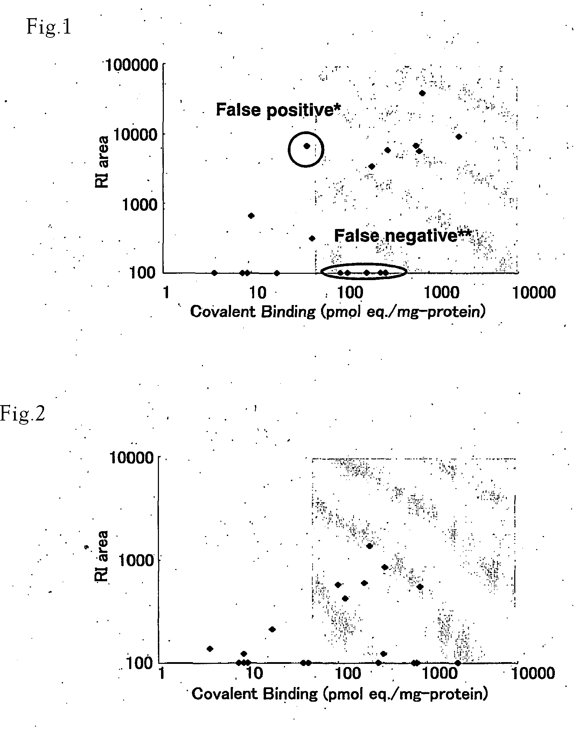

The results of the test in which [³⁵S]cysteine was used alone are shown in Fig. 1 by plotting covalent binding values obtained by a Brandel tissue harvester method on the horizontal axis and RI areas obtained by the assay on the vertical axis using a logarithmic scale. As shown in Fig. 1, a correlation was observed between the covalent binding value and the RI area value to some extent. However, a "false positive" compound, in which an RI area was observed despite of the absence of covalent binding, was observed. In addition, "false negative" compounds, which are the contrary phenomena, were also observed. On the other hand, as shown in Fig. 2, when sodium [¹⁴C]cyanide was used alone, the RI area showed a correlation with covalent binding to some extent in a group of compounds having a structure that can generate iminium, which is a hard electrophile. However, in a group of compounds having a structure that cannot generate iminium, RI area could not be obtained and thereby many false negatives occurred. Detailed analysis of these assay results revealed that compounds that did not generate RI peaks in the radio labeled cysteine system generated RI peaks in the radio labeled cyanide system.

### Example 2

Then, it was thought that, if a mixture of radiolabeled cysteine and cyanide was incubated, the results of two types of trapping agents may compensate each other by simultaneously trapping reactive metabolites produced through different two metabolic activation mechanisms, and "mixed trapping" using a mixture of two types of trapping agents was conducted.

First, test compound A and liver microsomes were incubated as in Example 1. One hour after the start of the incubation, acetonitrile (450 µL: a volume three times the volume of the reaction solution) was added to the reaction solution to terminate the reaction. Then, 150 µL of a phosphate buffer solution (1 M) was added to the solution. The resulting mixture was sufficiently mixed at room temperature and was then subjected to centrifugation at 10000 g for 5 minutes at room temperature to give an acetonitrile layer. Liquid layer separation was performed again by adding 450 µL of acetonitrile as in above method to give an acetonitrile layer. The resulting acetonitrile solution was concentrated and dried under nitrogen stream, and the residue was resuspended in 40 µL of a 40% methanol aqueous solution for being subjected to RI-HPLC analysis. The HPLC was conducted under the same conditions as in Example 1.

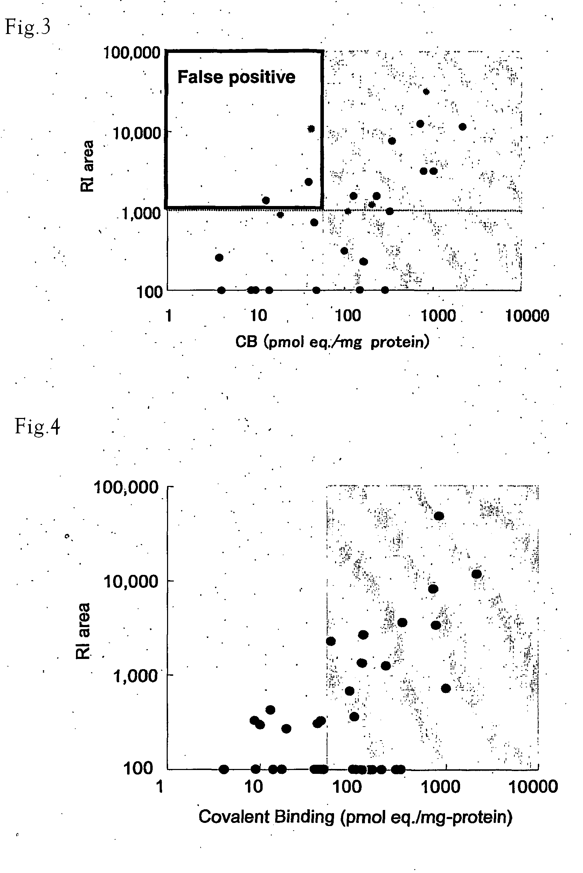

As shown in Fig. 3, the results of the mixed trapping confirmed improvement in a correlation between the RI area and the covalent binding, in particular, in frequency of false negative being observed, compared to the results when cysteine or sodium cyanide was used alone as the trapping agent. However, false positive was still detected in some specific compounds.

### Example 3

Almost all of the compounds that showed false positive in Example 2 had peaks based on radiolabeled cysteine. In addition, analysis of MS/MS fragment patterns in the LC-MS suggested a possibility that the binding patterns of the compounds were similar to those of cysteine. On the basis of this result, typical compounds that showed false positive were incubated with liver microsomes in the co-presence of cysteine. The resulting cysteine adducts were subjected to structural analysis using NMR. As a result, it was revealed that, in the cysteine adducts, cysteine is cyclized into a stable thiazolidine structure in the molecule. It was thought from the mechanism of producing a thiazolidine structure that the amino group of cysteine reacts with aldehyde produced by metabolism to produce imine, and the sulfhydryl group of cysteine reacts with the resulting imine to produce the thiazolidine structure. Accordingly, it was thought that this addition-cyclization reaction cannot be avoided as long as using cysteine as a trapping agent and possibility of false positive is high. Here, the production of aldehyde by metabolism cannot be avoided. Therefore, from the viewpoint of decreasing the RI peak by decomposing the produced thiazolidine or substituting radio labeled cysteine with unlabeled cysteine, sample treatment after incubation was investigated.

First, test compound A and liver microsomes were incubated as in Example 1. One hour after the start of the incubation, acetonitrile (450 µL: a volume three times the volume of the reaction solution) was added to the reaction solution to terminate the reaction. Then, 150 µL of a 1 M ammonium acetate aqueous solution was added to the solution. The resulting mixture was sufficiently mixed at room temperature and was then subjected to centrifugation at 10000 g for 5 minutes at room temperature to give an acetonitrile layer. Liquid-liquid extraction was performed again by adding 450 µL of acetonitrile as in above method to give an acetonitrile layer. Then, 150 µL of a 50 mM non-radio labeled cysteine aqueous solution was added to the resulting acetonitrile solution, and the mixture was sufficiently mixed and then incubated at 37°C for 5 hours. After the incubation, the solution was concentrated and dried under nitrogen stream, and the residue was resuspended in 40 µL of a 40% methanol aqueous solution for being subjected to RI-HPLC analysis. The HPLC was conducted under the same conditions as in Example 1.

As shown in Fig. 4, a correlation between the covalent binding and the RI area was improved. In addition, generation of adducts that cause false positive could be prevented. This result confirmed that the method of detecting a reactive metabolite according to the present invention is significantly useful as an assay of covalent binding.

### Example 4

An assay was performed as in Example 3 except that rat liver microsomes were used instead of human liver microsomes. The results showed a relatively favorable correlation between the covalent binding and the assay result, as in Example 3. Furthermore, generation of adducts that cause false positive was prevented. Therefore, it was confirmed that the method was significantly useful as an assay of covalent binding.

### Example 5

The following assay was conducted for simultaneously performing qualitative evaluation of an active metabolite by RI-HPLC-MS measurement when production of the active metabolite was quantitatively evaluated by RI-HPLC measurement.

### Incubation

A phosphate buffer solution (100 mM, pH 7.4) containing liver microsomes (1 mg/mL), 3 mM of magnesium chloride, 50 µM of a test compound (Troglitazone: SIGMA), and 1 mM of [³⁵S]cysteine or 100 µM of sodium [¹⁴C]cyanide was preinculated at 37°C for 5 minutes, and then an NADPH generating system was added therein to start the reaction (the concentration was the final concentration, and the incubation volume was 150 µL). The structural formula of Troglitazone is as follows:

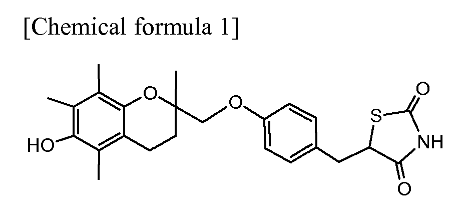

One hour later, acetonitrile (450 µL: a volume three times the volume of the reaction solution) was added to the reaction solution to terminate the reaction, and then 150 µL of an ammonium acetate aqueous solution (1 M) was added thereto. The resulting mixture was sufficiently mixed at room temperature and was then subjected to centrifugation at 13000 g for 5 minutes at room temperature to give an acetonitrile layer. Furthermore, 450 µL of acetonitrile was added to the acetonitrile layer, and the mixture was subjected to the same extraction as above to give an acetonitrile layer. Then, 150 µL of a 50 mM non-radio labeled cysteine aqueous solution was added to the resulting acetonitrile solution, and the mixture was sufficiently mixed and then incubated at 37°C for 5 hours. After the incubation, the solution was concentrated and dried under nitrogen stream, and the residue was resuspended in 40 µL of a 40% methanol aqueous solution. The suspension was subjected to RI-HPLC-MS analysis.

### Analysis method

The prepared sample was measured by RI-HPLC-MS at a sample injection volume of 30 µL. To prevent an RI-detector cell from being contaminated with foreign substances, the elution solution from the HPLC column was not introduced to a detector for 6.5 minutes after the sample injection (during that time, 0.1% formic acid-containing acetonitrile was introduced to the RI detector). The elution solution, after 6.5 minutes from the sample injection, was introduced to the RI detector and measured for radioactivity by online processing. Furthermore, the elution solution, after 7 minutes from the sample injection, was also introduced to a mass spectrometer. Thus, MS/MS(/MS) analysis was conducted simultaneously with the measurement of radioactivity using the RI detector. The ratio of the elution solution introduced to the mass spectrometer and the RI detector was mass spectrometer : RI detector = 1:4 (volume ratio).

In this assay, an ion trap mass spectrometer (LCQ deca XP plus: Thermoelectrone) was used. The MS/MS analysis was performed for [test compound molecular ion + 119] Da, which means addition of one molecule of cysteine to a test compound, and for [test compound molecular ion + 16 + 119] Da, which means addition of monooxidation and one molecule of cysteine to a test compound. In the MS/MS/MS analysis, ions that showed the highest intensity in an MS/MS fragment obtained in the MS/MS analysis were automatically subjected to MS/MS/MS scanning (dependent mass scan: a function attached to LCQ). A tuning file for MS(/MS) measurement of each compound was created before the measurement and was used in actual measurement.

The mobile phase was HCOOH/H₂O/100 mM AcONH₄/CH₃CN = 0.5/800/100/100 (solution A) and HCOOH/H₂O/100 mM AcONH₄/CH₃CN = 0.5/100/900 (solution B).
Gradient analysis was conducted for 70 minutes. The conditions for HPLC were as follows:
Model: Agilent1100 series
Column temperature: 40°C
Mobile phase:
   Binary pump A: CH₃CN/100 mM AcONH₄/H₂O/HCOOH = 100/100/800/0.5 (V/V/V/V)
   Binary pump B: CH₃CN/100 mM AcONH₄/HCOOH = 900/100/0.5 (V/V/V)
   Quarternary pump: CH₃CN/HCOOH = 100/0.1 (V/V)

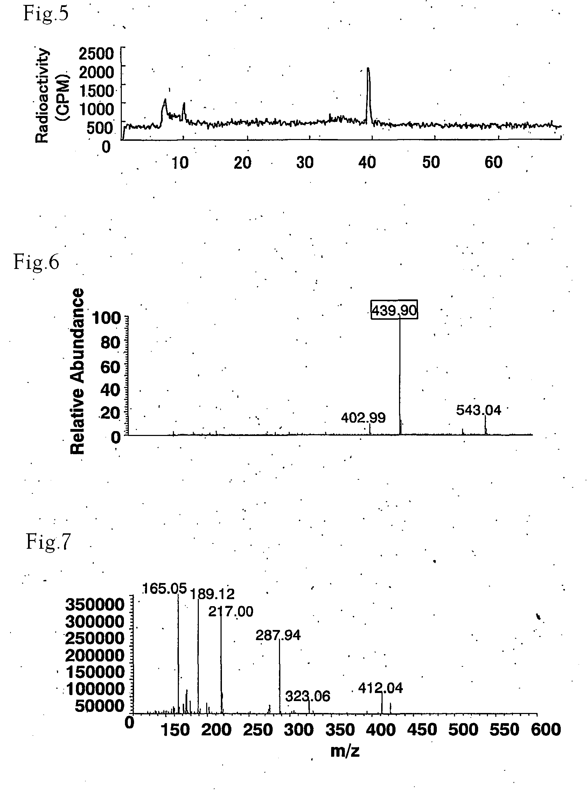

As shown in Figs. 5 to 7, in the MS/MS measurement, the MS/MS spectrum (Fig. 6) and the MS/MS/MS spectrum (Fig. 7) of a [³⁵S]cysteine adduct (Fig. 5) produced by incubation can be obtained by a single measurement. Thus, structural analysis of the adduct could be efficiently performed. The structural formula after metabolic reaction deduced from the structural analysis is shown in chemical formula 2.

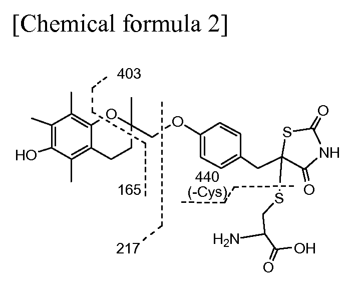

Since qualitative analysis of a compound becomes possible, the assay can be used as an indicator of structural conversion in compound synthesis and can also predict the position of a reactive metabolite produced in a compound.

That is, the method according to the present invention can quantitatively evaluate the production of a reactive metabolite and, at the same time, can narrow down the range of positions of metabolic activation. Thus, the method is effective for optimizing a compound in a drug discovery approach.

### Industrial Applicability

According to the method of detecting a reactive metabolite of the present invention, a reaction system can be simply prepared, and a reactive metabolite can be quantitatively detected. In addition, the method can qualitatively detect the reactive metabolite in a combination with MS. Furthermore, generation of a false positive signal can be prevented, and generation of a false negative signal can be decreased. Accordingly, pharmacokinetics investigation or screening of a test compound can be efficiently performed with higher accuracy. Therefore, the method can be used as a very convenient method in a drug development approach, drugs for not only human but also animals.

## Claims

1. A method of detecting a reactive metabolite, comprising steps of:
conducting a metabolic reaction of a test compound in the presence of two labeled nucleophiles; and
detecting the label of the nucleophiles bound to the reactive metabolite.

2. A method of detecting a reactive metabolite, comprising steps of:
conducting a metabolic reaction of a test compound in the presence of two labeled nucleophiles;
separating liquid layer using a high-concentration salt solution to obtain an organic layer; and
detecting the label of the nucleophiles bound to the reactive metabolite contained in the organic layer.

3. The method of detecting a reactive metabolite according to Claim 2, wherein the high-concentration salt is ammonium acetate.

4. The method of detecting a reactive metabolite according to Claim 2 or 3, further comprising mixing unlabeled cysteine with the organic layer obtained in the separation step and incubating the mixture.

5. The method of detecting a reactive metabolite according to any one of Claims 1 to 4, wherein the two nucleophiles are a soft nucleophile and a hard nucleophile.

6. The method of detecting a reactive metabolite according to any one of Claims 1 to 5, wherein the soft nucleophile is cysteine.

7. The method of detecting a reactive metabolite according to any one of Claims 1 to 6, wherein the hard nucleophile is cyanide.

8. The method of detecting a reactive metabolite according to any one of Claims 1 to 7, wherein the label is a radioisotope.

9. A method of detecting a reactive metabolite, comprising steps of:
conducting a metabolic reaction of a test compound in the presence of labeled cysteine; and
detecting the label of the nucleophile bound to the reactive metabolite.

10. A method of detecting a reactive metabolite, comprising steps of:
conducting a metabolic reaction of a test compound in the presence of labeled cysteine; separating liquid layer using a high-concentration salt solution to obtain an organic layer; and
detecting the label of the nucleophile bound to the reactive metabolite contained in the organic layer.

11. The method of detecting a reactive metabolite according to Claim 10, wherein the high-concentration salt is ammonium acetate.

12. The method of detecting a reactive metabolite according to Claim 10 or 11, further comprising mixing unlabeled cysteine with the organic layer obtained in the separation step and incubating the mixture.
